(19) Europäisches Patentamt
European Patent Office
Office européen des brevets

(11) **EP 3 231 417 B1**

(12) **EUROPÄISCHE PATENTSCHRIFT**

(45) Veröffentlichungstag und Bekanntmachung des Hinweises auf die Patenterteilung:
**05.12.2018 Patentblatt 2018/49**

(21) Anmeldenummer: **16165546.9**

(22) Anmeldetag: **15.04.2016**

(51) Int Cl.:
*A61K 9/00* (2006.01)     *A61K 47/36* (2006.01)
*A61K 31/728* (2006.01)

(54) **LAGERUNGSSTABILE, OPHTHALMISCHE ZUSAMMENSETZUNG**

STORAGE STABLE, OPHTHALMIC COMPOUND

COMPOSITION OPHTALMIQUE STABLE A L'ENTREPOSAGE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**18.10.2017 Patentblatt 2017/42**

(73) Patentinhaber: **Dr. Gerhard Mann Chem.-Pharm. Fabrik GmbH**
**13581 Berlin (DE)**

(72) Erfinder:
• **BELLMANN, Günther**
**13593 Berlin (DE)**
• **KRÖHNE, Lutz**
**13156 Berlin (DE)**

(74) Vertreter: **Müller-Dyck, Martina**
**Maiwald Patentanwalts- und Rechtsanwaltsgesellschaft mbH**
**Elisenhof**
**Elisenstraße 3**
**80335 München (DE)**

(56) Entgegenhaltungen:
**EP-A1- 1 913 934     WO-A1-2007/012625**
**US-A1- 2013 210 912**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft eine tropfbare, lagerungsstabile, ophthalmische Zusammensetzung sowie deren Herstellung und Verwendung.

Es ist allgemein bekannt, dass die Augen beispielsweise durch intensive Bildschirmarbeit, das Tragen von Kontaktlinsen oder durch trockene Raumluft beispielsweise durch Klimaanlagen häufig brennen, jucken oder tränen. Hierfür ist oft eine Funktionsstörung des Tränenfilms die Ursache, ausgelöst durch zu hohe Verdunstung oder zu geringe Tränenproduktion. Aber auch durch hormonelle Umstellungen im Alter, durch Einnahme von Medikamenten oder durch innere Erkrankungen wie dem Sjögren-Syndrom, Rheuma oder Diabetes können Benetzungsstörungen des Auges begünstigt werden.

Es ist im Stand der Technik bekannt, bei gereizten oder entzündeten Augen, insbesondere bei "trockenem Auge", *Keratokonjunktivitis sicca,* wässrige Präparate zur Behandlung der Beschwerden einzusetzen. Diese Präparate liegen üblicherweise in Form von Flüssigkeiten, Gelen, oder Cremes vor.

Nachteilig ist, dass Lösungen vielfach Inhaltsstoffe zugesetzt sind, die Allergien auslösen, zu Irritation führen oder Unverträglichkeiten gegenüber den Inhaltsstoffen erzeugen können. Auch ist von wässrigen Lösungen bekannt, dass diese häufig Jucken, Brennen oder andere unangenehme Nebenwirkungen beim Kontakt mit dem Auge erzeugen können. Weiterhin haben insbesondere Präparate in Form von Gelen, Cremes oder Salben den Nachteil, dass das Auftragen von den Patienten, die bereits an einer schmerzhaften Reizung des Auges leiden, als zusätzlich unangenehm empfunden wird. Hinzu kommt, dass die Verteilung des Präparates im oder am Auge häufig durch Reiben unterstützt werden muss, ein Vorgang, der ebenfalls zu einem zusätzlichen Schmerzempfinden bei dem Patienten führt.

Es sind auch zweiphasige Emulsionen im Stand der Technik bekannt. Deren Stabilität ist unter anderem jedoch von ihrer Viskosität abhängig. So werden Emulsionen beispielweise instabil, wenn eine in einer hydrophilen Phase feindispergierte hydrophobe Phase aggregiert. Dieser Vorgang schränkt die Stabilität bei einer Lagerung der Emulsion ein und läuft umso langsamer ab, je viskoser die kontinuierliche Phase der Emulsion ist. Um eine ausreichende Stabilität von Emulsionen erreichen zu können, werden üblicherweise Emulgatoren eingesetzt. Emulgatoren können jedoch ebenfalls allergische oder auf Überempfindlichkeit beruhende Reaktionen hervorrufen.

Ein emulgatorfreies Präparat ist beispielsweise in EP 1913934 A1 offenbart. Dieses Präparat hat jedoch den Nachteil, dass das Präparat eine hohe Viskosität aufweist, so dass die Applikation des Präparates für den Patienten unangenehm sein kann und eine Unterstützung der Verteilung des Präparates beispielsweise durch Reiben im Auge zusätzliche Schmerzen auslösen kann. Weiterhin ist die Entnahme von höherviskose Präparaten aus Ophtiolen oder Ein-Dosis-Ophtiolen schwierig, insbesondere für ältere Patienten oder Patienten mit eingeschränkten motorischen Fähigkeiten.

WO 2007/012625 A1 beschreibt ein steriles, tropfbares, mehrphasiges, emulgatorfreies, ophthalmisches Präparat mit einer Viskosität im Bereich von größer oder gleich 200 mPa·s und weniger als 2000 mPa·s. Allerdings werden bei dieser Formulierung Phosphatsalze eingesetzt, die in seltenen Fällen bei Patienten mit signifikanter Schädigung der Hornhautoberfläche mit dem im Hornhautstroma vorhandenen Kalzium reagieren und zur Ausfällung von Kalziumphosphat auf der Hornhaut führen können (siehe BfArM & PEI Bulletin zur Arzneimittelsicherheit 2013, 1, 7-12).

Lagerungsstabile, mehrphasige ophthalmische Zusammensetzungen werden auch in WO 2013/122801 A1 beschrieben. Allerdings können diese Zusammensetzungen Emulgatoren, Puffer oder auch Phosphatsalze enthalten.

Es besteht somit weiterhin ein Bedarf im Stand der Technik an lagerungsstabilen ophthalmischen Zusammensetzungen, die sehr gut verträglich sind und die bekannten Nachteile vermeiden.

Es ist daher Aufgabe der vorliegenden Erfindung eine ophthalmische Zusammensetzung bereitzustellen, die lagerungsstabil und sehr gut verträglich ist. Es ist wünschenswert, dass die Zusammensetzung tropfbar ist und eine lipophile Komponente enthält. Des Weiteren ist es wünschenswert, dass die Zusammensetzung auch bei einer geringen Viskosität ohne Zusatz von Emulgatoren oder Puffersubstanzen lagerungsstabil ist.

Es ist weiterhin eine Aufgabe der vorliegenden Erfindung eine ophthalmische Zusammensetzung zur Verwendung als Arzneimittel bereitzustellen. Es ist wünschenswert, dass sich diese Zusammensetzung zur Behandlung von Krankheiten und Fehlfunktionen des Auges oder der das Auge umgebenden oder damit zusammenhängenden Organe und Gewebe eignet, insbesondere zur Behandlung des trockenen Auges.

Die zuvor genannten und weitere Aufgaben werden gemäß den in den unabhängigen Ansprüchen definierten Gegenständen gelöst.

Gemäß einem Aspekt der vorliegenden Erfindung wird eine lagerungsstabile, mehrphasige, ophthalmische Zusammensetzung bereitgestellt, enthaltend mindestens eine flüssige wässrige Phase und mindestens eine flüssige hydrophobe Phase, dadurch gekennzeichnet, dass

die Zusammensetzung emulgatorfrei ist,
die Zusammensetzung pufferfrei ist, und
die Zusammensetzung Hyaluronsäure und/oder Hyaluronat enthält.

Gemäß einem weiteren Aspekt der vorliegenden Erfindung wird ein Verfahren zur Herstellung einer tropfbaren, lagerungsstabilen, mehrphasigen, ophthalmischen Zusammensetzung gemäß der vorliegenden Erfindung bereitgestellt, dadurch gekennzeichnet, dass eine flüssige hydrophobe Phase homogen in einer kontinuierlichen flüssigen wässrigen Phase dispergiert wird, wobei die flüssige wässrige Phase Hyaluronsäure und/oder Hyaluronat umfasst.

Gemäß einem weiteren Aspekt der vorliegenden Erfindung wird eine tropfbare, lagerungsstabile, mehrphasige, ophthalmische Zusammensetzung gemäß der vorliegenden Erfindung, zur Verwendung als Arzneimittel bereitgestellt.

Gemäß einem weiteren Aspekt der vorliegenden Erfindung wird eine tropfbare, lagerungsstabile, mehrphasige, ophthalmische Zusammensetzung gemäß der vorliegenden Erfindung, zur Verwendung bei der Behandlung von Krankheiten und Fehlfunktionen des Auges oder der das Auge umgebenden oder damit zusammenhängenden Organe und Gewebe bereitgestellt, bevorzugt zur Verwendung bei der Behandlung des trockenen Auges.

Gemäß einem weiteren Aspekt der vorliegenden Erfindung wird ein Behältnis enthaltend eine Zusammensetzung gemäß der vorliegenden Erfindung bereitgestellt, wobei das Behältnis ein Einmaldosen-Behältnis oder ein Mehrdosen-Behältnis ist, bevorzugt eine Ophtiole, eine Ein-Dosis-Ophtiole, oder ein Applikationssystem, bevorzugter ein pumpenbasiertes Applikationssystem oder ein Tip-Seal-Applikationssystem.

Gemäß einem weiteren Aspekt der vorliegenden Erfindung wird die Verwendung von Hyaluronsäure und/oder Hyaluronat zur Stabilisierung einer mehrphasigen, emulgatorfreien Zusammensetzung bereitgestellt, umfassend mindestens eine flüssige wässrige Phase und mindestens eine flüssige hydrophobe Phase.

Weitere vorteilhafte Ausführungsformen der vorliegenden Erfindung sind in den entsprechenden Unteransprüchen beschrieben.

Gemäß einer Ausführungsform weist die Zusammensetzung eine Viskosität von weniger als 350 mPa·s bei 20°C auf, bevorzugt weniger als 250 mPa·s bei 20°C, bevorzugter weniger als 200 mPa·s bei 20°C, noch bevorzugter weniger als 150 mPa·s bei 20°C, und am meisten bevorzugt weniger als 100 mPa·s bei 20°C. Gemäß einer weiteren Ausführungsform enthält die Zusammensetzung Hyaluronsäure und/oder Hyaluronat in einer Menge von mindestens 0,001 Gew.-%, bezogen auf das Gesamtgewicht der Zusammensetzung, bevorzugt in einer Menge von mindestens 0,01 Gew.-%, bevorzugter in einer Menge von mindestens 0,1 Gew.-%, noch bevorzugter in einer Menge von mindestens 0,15 Gew.-%, und am meisten bevorzugt in einer Menge von mindestens 0,2 Gew.-%.

Gemäß einer Ausführungsform weist die Hyaluronsäure und/oder das Hyaluronat ein Molekulargewicht $M_w$ von 50 000 bis 10 000 000 g/mol auf, bevorzugt von 100 000 bis 5 000 000 g/mol, und am meisten bevorzugt von 250 000 bis 1 000 000 g/mol. Gemäß einer weiteren Ausführungsform ist das Hyaluronat ausgewählt ist aus der Gruppe bestehend aus Natrium-Hyaluronat, Kalium-Hyaluronat, Zink-Hyaluronat und Mischungen davon, bevorzugt ist das Hyaluronat Natrium-Hyaluronat. Gemäß einer weiteren Ausführungsform enthält die Zusammensetzung die flüssige wässrige Phase als kontinuierliche Phase und die flüssige hydrophobe Phase als darin dispergierte Tröpfchen.

Gemäß einer Ausführungsform umfasst die Zusammensetzung mindestens eine polymere, gelbildende Komponente, bevorzugt mindestens eine Polyacrylsäure und/oder mindestens ein polymeres Acrylsäurederivat, und am meisten bevorzugt mindestens ein Carbomer. Gemäß einer weiteren Ausführungsform umfasst die flüssige hydrophobe Phase ein ophthalmisch verträgliches Öl, bevorzugt ein Triglycerid, und am meisten bevorzugt ein mittelkettiges Triglycerid. Gemäß einer weiteren Ausführungsform umfasst die Zusammensetzung ein Mittel zur Einstellung der Isotonizität, bevorzugt ausgewählt aus der Gruppe bestehend aus Dextrose, Glycerin, Propylenglycol, Sorbitol, Mannitol, Harnstoff, Polyethylenglycol, Borsäure, Magnesiumsulfat, Zinksulfat, Natriumchlorid, Kaliumchlorid, Calciumchlorid, Natriumsulfat, Natriumhydrogenphosphat, Trinatriumcitrat, Trinatriumphosphat, und Mischungen davon, bevorzugter ausgewählt aus der Gruppe bestehend aus Dextrose, Glycerin, Propylenglycol, Sorbitol, Mannitol, Harnstoff, Polyethylenglycol, und am meisten bevorzugt Glycerin.

Gemäß einer Ausführungsform umfasst die Zusammensetzung, bezogen auf das Gesamtgewicht der Zusammensetzung, die folgenden Stoffe:

0,15 bis 0,3 Gew.-% Natrium-Hyaluronat,
0,15 bis 0,25 Gew.-% mittelkettige Triglyceride,
0,05 bis 0,1 Gew.-% Carbomer,
1,5 bis 7 Gew.-% Mittel zur Einstellung der Isotonizität, bevorzugt Glycerin, Natriumhydroxid zur pH Einstellung, und
q.s. ad 100 Gew.-% Wasser.

Nachfolgend werden in der vorliegenden Anmeldung verwendete Begriffe näher erläutert:
Im Sinne der vorliegenden Erfindung bedeutet der Begriff "tropfbar", dass die ophthalmische Zusammensetzung fähig ist Tropfen zu bilden und sich daher in Form von Tropfen applizieren lässt. Die Tropfengröße ist abhängig von dem Behältnis und insbesondere dem Tropfer, aus dem die Zusammensetzung appliziert wird. Zum Beispiel kann die Tropfengröße in einem Bereich von 5 bis 70 µl liegen.

Der Begriff "lagerungsstabil" bedeutet im Rahmen der vorliegenden Erfindung, dass keine sichtbare Phasenseparation auftritt und die Zusammensetzung kaum oder nur geringfügige Veränderungen im Hinblick auf pH-Wert, Osmolalität, Viskosität und Aussehen zeigt, wenn sie über einen Zeitraum von mindestens 12 Monaten bei einer Temperatur von 2 bis 8°C gelagert wird. Gemäß einer Ausführungsform ist eine Zusammensetzung lagerungsstabil, wenn ein Tropfen, bevorzugt 50 μl, der Zusammensetzung, nachdem sie über einen Zeitraum von 12 Monaten bei einer Temperatur von 2 bis 8°C gelagert wurde, maximal 10 Öltropfen (lipophile Phase) mit einem Tropfendurchmesser von mehr als 150 μm und maximal 15 Öltropfen (lipophile Phase) mit einem Durchmesser von mehr als 100 μm aufweisen. Der Tropfendurchmesser der lipophilen Phase kann mikroskopisch ermittelt werden.

"Emulgatorfrei" bedeutet im Sinne dieser Erfindung, dass die Zusammensetzung ausschließlich Inhaltsstoffe aufweist, die keine Emulgatoren im Sinne der vorliegenden Erfindung sind. "Emulgatoren" im Sinne der vorliegenden Erfindung sind grenzflächenaktive Substanzen, die in der Lage sind, die Grenzflächenspannung zwischen Öl und Wasserphase zu vermindern, indem sie sich bevorzugt an der Grenzfläche zwischen diesen beiden anlagern. Dies wird durch den amphiphilen Molekülaufbau der Emulgatoren ermöglicht, die wenigstens eine polare (hydrophile) Gruppe und wenigstens eine unpolare (lipophile) Gruppe besitzen. Damit sind sie sowohl in der hydrophilen als auch in der lipophilen Phase löslich. Der in der entsprechenden Phase besser lösliche Teil ragt in diese hinein und senkt dadurch die Grenzflächenspannung zwischen beiden Phasen.

Insbesondere kann die Zusammensetzung gemäß der vorliegenden Erfindung Stoffe ausgewählt aus der Gruppe bestehend aus einem ophthalmisch verträglichem Öl, eine polymere, gelbildende Komponente, ein Mittel zur Einstellung der Isotonizität, einen ophthalmischen Wirkstoff, Säuren oder Basen zur Einstellung des pH-Wertes, Wasser und Mischungen davon aufweisen, wobei es sich bei diesen Stoffen im Sinne der vorliegenden Erfindung nicht um Emulgatoren handelt.

"Pufferfrei" bedeutet im Sinne dieser Erfindung, dass die Zusammensetzung ausschließlich Inhaltsstoffe aufweist, die keine Puffer im Sinne der vorliegenden Erfindung sind. Ein "Puffer" im Sinne der vorliegenden Erfindung kann ein Puffersystem sein, z.B. eine Mischung aus einer Säure und ihrer konjugierten Base, oder eine Puffersubstanz. Beispiele für Puffer im Sinne der vorliegenden Erfindung sind Phosphat-Puffer, Phosphat-Citrat-Puffer, Citrat-Puffer, Tartrat-Puffer, Borat-Puffer, Malat-Puffer, Succinat-Puffer, Acetat-Puffer, Acetat-Borat-Puffer, MES (2-(N-Morpholino)ethansulfsäure), HEPES (2-(4-(2-Hydroxyethyl)-1-piperazinyl)-ethansulfonsäure), BES (N,N-Bis(2- hydroxyethyl)-2-aminoethansulfonsäure), MOPS (3-($N$-Morpholino)-propansulfonsäure), TRIS (Tris(hydroxymethyl)-aminomethan), oder BIS-TRIS (Bis(2-hydroxyethyl)amino-tris(hydroxymethyl)methan).

Gemäß einer Ausführungsform ist die Zusammensetzung pufferfrei, wenn die Pufferkapazität $P_i$ der Zusammensetzung weniger als 0,005 beträgt, und bevorzugt weniger als 0,0005 beträgt. Zur Bestimmung der Pufferkapazität $P_i$ wird zu 10 ml der zu prüfenden Zusammensetzung so viel 0,1 N Natronlauge gegeben, bis eine pH-Verschiebung von 0,3 Einheiten eintritt. Die Pufferkapazität $P_i$ lässt sich dann wie folgt bestimmen:

$$P_i = \frac{\text{ml Lauge} \cdot \mathbf{0,1}}{\text{pH-Verschiebung} \cdot \mathbf{10}}$$

(Vgl. Dolder, Skinner, Ophthalmika, 4. Auflage, Wissenschaftliche Verlagsgesellschaft, Seite 606, Kapitel 4.1.2.4).

Unter dem Begriff "Viskosität" wird vorliegend, sofern nicht anders angegeben, die dynamische Viskosität verstanden, die auch als absolute Viskosität bezeichnet wird. Gemäß der vorliegenden Erfindung wird die Viskosität nach dem Kegel-Platte-Verfahren unter Verwendung eines Wells-Brookfield Kegel-Platte-Viskosimeters des Typs DV-III+ (Brookfield Engineering Laboratories Vertriebs GmbH, Deutschland) mit einer CP51 Spindel bei 5 U/min und 20°C bestimmt. Die Viskosität bezieht sich, sofern nicht anders angegeben, auf die Viskosität der Zusammensetzung vor der Applikation im Auge.

Sofern ein unbestimmter oder bestimmter Artikel im Zusammenhange mit einem Substantiv im Singular verwendet wird, wie z.B. "ein/eine/einer" oder "die/der/das", schließt dies auch immer den Plural dieses Substantivs mit ein, es sei denn es ist etwas anderes angegeben.

Wenn der Begriff "umfassend" in der vorliegenden Beschreibung oder Ansprüchen, verwendet wird, schließt dies nicht andere Elemente aus. Im Sinne der vorliegenden Erfindung, wird der Begriff "bestehend aus" als eine bevorzugte Ausführungsform des Begriffs "umfassend" verstanden. Wird nachstehend eine Gruppe von Elementen definiert, die mindestens eine bestimmte Zahl von Ausführungsformen umfasst, ist dies so zu verstehen, dass die Gruppe vorzugsweise nur aus diesen Elementen besteht.

Begriffe wie "erhältlich" oder "definierbar" und "erhalten" oder "definiert" werden synonym verwendet. Dies bedeutet zum Beispiel, sofern sich aus dem Zusammenhang nichts anderes ergibt, dass der Begriff "erhalten" nicht so zu verstehen ist, dass beispielsweise eine Ausführungsform z.B. durch eine bestimmte Schrittreihenfolge erhalten werden muss, die dem Begriff "erhalten" folgt. Nichtsdestotrotz beinhalten die Begriffe "erhalten" oder "definiert" diese eingeschränkte Betrachtung als bevorzugte Ausführungsform.

Wann auch immer die Begriffe "einschließend" oder "aufweisend" verwendet werden, sind diese als äquivalent zu dem Begriff "umfassend" anzusehen.

Gemäß der vorliegenden Erfindung wird eine tropfbare, lagerungsstabile, mehrphasige, ophthalmische Zusammensetzung bereitgestellt, die mindestens eine flüssige wässrige Phase und mindestens eine flüssige hydrophobe Phase beinhaltet. Die Zusammensetzung ist, dadurch gekennzeichnet, dass sie sowohl emulgatorfrei als auch pufferfrei ist und Hyaluronsäure und/oder Hyaluronat enthält.

Nachfolgend werden bevorzugte Ausführungsformen der vorliegenden Erfindung ausführlich beschrieben. Diese Ausführungsformen und Angaben gelten entsprechend für das erfindungsgemäße Verfahren und die erfindungsgemäßen Verwendungen.

Die flüssige wässrige und die flüssige hydrophobe Phase

Die ophthalmische Zusammensetzung der vorliegenden Erfindung ist mehrphasig und enthält mindestens eine flüssige wässrige Phase und mindestens eine flüssige hydrophobe Phase. Die hydrophobe Phase kann auch als lipophile Phase bezeichnet werden.

Gemäß einer Ausführungsform umfasst die flüssige wässrige Phase Wasser, und bevorzugt Wasser für Injektionszwecke. Gemäß einer anderen Ausführungsform bildet die flüssige wässrige Phase den natürlichen Tränenfilm nach. Hierdurch kann die flüssige wässrige Phase die Hornhaut befeuchten und eine reinigende und schützende Funktion ausüben. Gemäß einer Ausführungsform weist die flüssige wässrige Phase einen pH-Wert von 7,1 bis 7,8 auf und/oder eine Osmolalität von 260 bis 320 mosmol/kg.

Gemäß einer Ausführungsform der vorliegenden Erfindung umfasst die flüssige hydrophobe Phase ein ophthalmisch verträgliches Öl. Gemäß einer Ausführungsform der vorliegenden Erfindung ist das ophthalmisch verträgliche Öl ausgewählt aus der Gruppe bestehend aus Fettsäureestern, Phthalsäureestern, mittelkettigen Triglyceriden, langkettigen Triglyceriden, Erdnussöl, Olivenöl, Kokosnussöl, Sesamöl, Baumwollsamenöl, Sonnenblumenöl, Maiskeimöl, Kiwisamenöl, Leinöl, Walnussöl, Rapsöl, Chaiöl, Perillaöl, Hanföl, Palmöl, Mandelöl, Fischöl, Algenöl und Mischungen davon.

Vorzugsweise ist das Öl ein biologisches Öl, d.h. ein Öl pflanzlichen oder tierischen Ursprungs. Gemäß einer Ausführungsform umfasst die hydrophobe Phase ein ophthalmisch verträgliches Pflanzenöl, vorzugsweise ausgewählt aus der Gruppe bestehend aus Erdnussöl, Olivenöl, Kokosnussöl, Sesamöl, Baumwollsamenöl, Sonnenblumenöl, Maiskeimöl, Kiwisamenöl, Leinöl, Walnussöl, Rapsöl, Chiaöl, Perillaöl, Hanföl, Palmöl, Mandelöl, und Mischungen davon.

"Mittelkettige Triglyceride" im Sinne der vorliegenden Erfindung weisen Fettsäuren mit einer Kettenlänge von 6 bis 12 C-Atomen auf. "Langkettige Triglyceride" im Sinne der vorliegenden Erfindung weisen Fettsäuren mit einer Kettenlänge von 14 bis 18 C-Atomen auf.

Gemäß einer Ausführungsform umfasst die flüssige hydrophobe Phase ein Triglycerid. Verwendbare Triglyceride sind erhältlich in Form von synthetischen, halbsynthetischen oder natürlichen Ölen, wie Olivenöl, Palmöl, Mandelöl, Kokosnussöl oder deren Mischungen. Bevorzugt verwendbare Triglyceride sind erhältlich aus Kokosnussöl. Solche mittelkettigen Triglyceride werden beispielsweise aus dem Öl des Endosperms von *Cocos nucifera L.,* vorzugsweise des festen getrockneten Teils, oder *Elasis guineensis Jacq.* hergestellt. Weiterhin bevorzugt verwendbare Triglyceride, vorzugsweise mittelkettige Triglyceride, sind Neutralöle, beispielsweise erhältlich unter den Handelsnamen Myritol® 312, Myritol® 318, oder Myritol® 331, kommerziell erhältlich von der Firma Cognis Ltd., Japan. Insbesondere sind auch mittelkettige Triglyceride der Art bevorzugt, wie sie im Europäischen Arzneibuch, European Pharmacopoeia 5.0, 01/2010:0868, S. 3471, *Triglycerida saturata media,* beschrieben werden. Bevorzugt enthält der Säurebestandteil ein Gemisch mit einem Anteil von wenigstens 90 Gew.-%, bevorzugter wenigstens 94 Gew.-%, am meisten bevorzugt wenigstens 95 Gew.-%, bezogen auf das Gesamtgewicht der Fettsäuren n-Octansäure und n-Decansäure. Andere geeignete, mittelkettige Triglyceride sind unter den Handelsnamen Acomed®, Captex®, Neobee®M5F, Miglyol®810, Miglyol®812, Mazol®, oder Sefsol®860 kommerziell erhältlich. Die mittelkettigen Triglyceride können vorteilhafter Weise die Verdunstung der wässrigen Komponente verhindern und einem zu raschen Trocknen des Tränenfilms oder der ophthalmischen Zusammensetzung vorbeugen.

Gemäß einer Ausführungsform ist das Triglycerid ein mittelkettiges Triglycerid. Das mittelkettige Triglycerid kann beispielsweise aus, bezogen auf das Gesamtgewicht der Fettsäuren, wenigstens 80 Gew.-%, bevorzugt wenigstens 90 Gew.-%, und bevorzugter wenigstens 95 Gew.-%, $C_8$-$C_{12}$-Fettsäuren ausgebildet sein, bevorzugt $C_8$-$C_{10}$-Fettsäuren. Vorzugsweise kann das mittelkettige Triglyceride ein gesättigtes mittelkettiges Triglycerids sein. Gemäß einer Ausführungsform ist das gesättigte mittelkettige Triglycerid ausgebildet aus wenigstens 80 Gew.-%, bevorzugt wenigstens 90 Gew.-%, und bevorzugter wenigstens 95 Gew.-%, bezogen auf das Gesamtgewicht der Fettsäuren, gesättigten $C_8$- und $C_{10}$-Fettsäuren.

Besonders bevorzugt sind Capryl-Caprinsäure-Triglyceride, d.h. eine Mischung ausgebildet aus veresterten gesättigten $C_8$- und $C_{10}$-Fettsäuren. Diese Mischung hat den Vorteil, dass die gesättigten Fettsäuren eine verbesserte Stabilität des Lipidfilms und damit eine überraschend verbesserte längere Verweildauer der Zusammensetzung im Auge zur Verfügung stellen können.

Gemäß einer Ausführungsform besteht die flüssige hydrophobe Phase aus einem ophthalmisch verträglichen Öl, bevorzugt ausgewählt aus der Gruppe bestehend aus Fettsäureestern, Phthalsäureestern, mittelkettigen Triglyceriden, langkettigen Triglyceriden, Erdnussöl, Olivenöl, Kokosnussöl, Sesamöl, Baumwollsamenöl, Sonnenblumenöl, Maiskeimöl, Kiwisamenöl, Leinöl, Walnussöl, Rapsöl, Chaiöl, Perillaöl, Hanföl, Palmöl, Mandelöl, Fischöl, Algenöl und Mischungen davon, bevorzugter ausgewählt aus der Gruppe bestehend aus Fettsäureestern, Phthalsäureestern, mittelkettigen Triglyceriden, langkettigen Triglyceriden, Erdnussöl, Olivenöl, Kokosnussöl, Sesamöl, Baumwollsamenöl, Sonnenblumenöl, Maiskeimöl, Kiwisamenöl, Leinöl, Walnussöl, Rapsöl, Chaiöl, Perillaöl, Hanföl, Palmöl, Mandelöl, und Mischungen davon, und am meisten bevorzugt ist das ophthalmisch verträgliche Öl ein mittelkettiges Triglycerid. Gemäß einer weiteren Ausführungsform umfasst die flüssige hydrophobe Phase ein ophthalmisch verträgliches Öl, bevorzugt ein Triglycerid, und am meisten bevorzugt ein mittelkettiges Triglycerid.

Gemäß einer Ausführungsform umfasst die Zusammensetzung das ophthalmisch verträgliche Öl in einer Menge von 0,05 bis 10 Gew.-%, bezogen auf das Gesamtgewicht der Zusammensetzung, bevorzugt von 0,1 bis 5 Gew.-%, bevorzugter von 0,15 bis 2 Gew.-%, und am meisten bevorzugt von 0,2 bis 1,5 Gew.-%. Gemäß einer bevorzugten Ausführungsform umfasst die Zusammensetzung das ophthalmisch verträgliche Öl in einer Menge von weniger als 0,5 Gew.-%, bezogen auf das Gesamtgewicht der Zusammensetzung. Die Erfinder der vorliegenden Erfindung haben herausgefunden, dass in der erfindungsgemäßen Zusammensetzung bereits eine geringe Menge an ophthalmisch verträglichem Öl nach dem Einbringen in das Auge eine ausreichende Verdunstungsbarriere bereitstellen kann. Hierdurch kann eine Sehbeeinträchtigung nach der Applikation der Zusammensetzung vermieden oder reduziert werden.

Gemäß einer Ausführungsform umfasst die Zusammensetzung das ophthalmisch verträgliches Öl in einer Menge von 0,05 bis 0,45 Gew.-%, bezogen auf das Gesamtgewicht der Zusammensetzung, bevorzugt von 0,1 bis 0,4 Gew.-%, bevorzugter von 0,15 bis 0,35 Gew.-%, und am meisten bevorzugt von 0,2 bis 0,3 Gew.-%. Beispielsweise kann die erfindungsgemäße Zusammensetzung das ophthalmisch verträgliche Öl in einer Menge von 0,15 bis 0,25 Gew.-%, bezogen auf das Gesamtgewicht der Zusammensetzung, umfassen.

Gemäß einer Ausführungsform enthält die mehrphasige, ophthalmische Zusammensetzung die flüssige wässrige Phase als kontinuierliche Phase, und die flüssige hydrophobe Phase als darin dispergierte Tröpfchen. Die Tröpfchen können einen Durchmesser von 1 bis 30 $\mu$m aufweisen, vorzugsweise von 5 bis 15 $\mu$m.

Das Präparat ist vorzugsweise wenigstens zweiphasig ausgebildet, und kann in bevorzugten Ausführungsformen auch dreiphasig ausgebildet sein. Die Zusammensetzung kann zum Beispiel eine zusätzliche wässrige Phase mit einer polymeren, gelbildenden Komponente enthalten, wie sie weiter unten beschrieben ist.

Die erfindungsgemäße Zusammensetzung kann einen hohen Anteil an flüssiger wässriger Phase aufweisen. Gemäß einer Ausführungsform umfasst die Zusammensetzung die flüssige wässrige Phase in einer Menge von 60 bis 99,95 Gew.-%, bezogen auf das Gesamtgewicht der Zusammensetzung, bevorzugt 80 bis 99,9 Gew.-%, bevorzugter von 90 bis 99,85 Gew.-%, und am meisten bevorzugt von 92 bis 99,8 Gew.-%. Gemäß einer anderen Ausführungsform umfasst Zusammensetzung die flüssige hydrophobe Phase in einer Menge von 0,05 bis 10 Gew.-%, bezogen auf das Gesamtgewicht der Zusammensetzung, bevorzugt von 0,1 bis 5 Gew.-%, bevorzugter von 0,15 bis 2 Gew.-%, und am meisten bevorzugt von 0,2 bis 1,5 Gew.-%. Gemäß einer bevorzugten Ausführungsform besteht die flüssige hydrophobe Phase vollständig aus dem ophthalmisch verträglichen Öl.

Hyaluronsäure und/oder Hyaluronat

Die ophthalmische Zusammensetzung der vorliegenden Erfindung ist dadurch gekennzeichnete, dass sie Hyaluronsäure und/oder Hyaluronat enthält.

Hyaluronsäure ist ein natürlich vorkommendes hochviskoses Glycosaminoglykan bestehend aus den Glucosederivaten D-Glucuronsäure und *N*-Acetyl-D-glucosamin. Je nach Extraktionsverfahren kann das Molekulargewicht $M_w$ der Hyaluronsäure in dem Bereich von 50 000 bis 10 000 000 g/mol liegen. Die Hyaluronsäure ist ein Bestandteil der extrazellulären Matrix von Wirbeltieren und insbesondere ein natürlicher Bestandteil der menschlichen Augenflüssigkeiten. Die Hyaluronsäure kann aus tierischen oder pflanzlichen Quellen gewonnen oder synthetisch oder halbsynthetisch hergestellt werden.

Gemäß einer Ausführungsform der vorliegenden Erfindung, enthält die Zusammensetzung Hyaluronsäure und/oder Hyaluronat in einer Menge von mindestens 0,001 Gew.-%, bezogen auf das Gesamtgewicht der Zusammensetzung, bevorzugt in einer Menge von mindestens 0,01 Gew.-%, bevorzugter in einer Menge von mindestens 0,1 Gew.-%, noch bevorzugter in einer Menge von mindestens 0,15 Gew.-%, und am meisten bevorzugt in einer Menge von mindestens 0,2 Gew.-%. Gemäß einer weiteren Ausführungsform der vorliegenden Erfindung, enthält die Zusammensetzung Hyaluronsäure und/oder Hyaluronat in einer Menge von 0,001 bis 5 Gew.-%, bezogen auf das Gesamtgewicht der Zusammensetzung, bevorzugt in einer Menge von 0,01 bis 2 Gew.-%, bevorzugter in einer Menge von 0,1 bis 1,5 Gew.-%, noch bevorzugter in einer Menge von 0,15 bis 1 Gew.-%, und am meisten bevorzugt in einer Menge von 0,2 bis 0,8 Gew.-%. Beispielsweise kann die erfindungsgemäße Zusammensetzung Hyaluronsäure und/oder Hyaluronat in einer Menge von 0,15 bis 0,3 Gew.-%, bezogen auf das Gesamtgewicht der Zusammensetzung, umfassen.

Gemäß einer Ausführungsform weist die Hyaluronsäure und/oder das Hyaluronat ein Molekulargewicht $M_w$

von 50 000 bis 10 000 000 g/mol auf, bevorzugt von 100 000 bis 5 000 000 g/mol, und am meisten bevorzugt von 250 000 bis 1 000 000 g/mol.

Statt der freien Hyaluronsäure oder zusätzlich zu der freien Hyaluronsäure kann in der ophthalmischen Zusammensetzung der vorliegenden Erfindung auch ein Salz der Hyaluronsäure, d.h. ein Hyaluronat, eingesetzt werden. Gemäß einer Ausführungsform ist das Hyaluronat ausgewählt aus der Gruppe bestehend aus Natrium-Hyaluronat, Kalium-Hyaluronat, Zink-Hyaluronat und Mischungen davon. Gemäß einer bevorzugten Ausführungsform ist das Hyaluronat Natrium-Hyaluronat.

Die Erfinder der vorliegenden Erfindung haben überraschenderweise herausgefunden, dass der Zusatz von Hyaluronsäure und/oder Hyaluronat die Stabilität und insbesondere die Lagerungsstabilität von mehrphasigen ophthalmischen Zusammensetzungen verbessert. Ohne an eine bestimmte Theorie gebunden zu sein, vermuten die Erfinder, dass die Hyaluronsäure und/oder das Hyaluronat die in der flüssigen wässrigen Phase dispergierten Tröpfchen der flüssigen hydrophoben Phase über einen sterischen Effekt stabilisieren kann.

Des Weiteren hat sich überraschend gezeigt, dass die erfindungsgemäße Zusammensetzung auch in niedrigen Viskositätsbereichen, insbesondere bei einer Viskosität von weniger als 350 mPa·s, z.B. bei weniger als 250 mPa·s oder weniger als 200 mPa·s, über einen längeren Zeitraum stabil bleibt. Dies kann insbesondere für die Formulierung von niedrigviskosen Emulsionen vorteilhaft sein, da diese üblicherweise bei Lagerung rasch instabil werden und sich entmischen. Insbesondere ist von Vorteil, dass eine Zusammensetzung mit geringer Viskosität die Applizierbarkeit für den Patienten deutlich verbessert. Somit kann in vorteilhafter Weise eine tropfbare Zusammensetzung zur Verfügung gestellt werden, die sich aufgrund der geringen Viskosität auf der Cornea des Auges besser verteilen kann, und das Auge weniger durch eine zusätzliche mechanische Unterstützung der Verteilung gereizt werden muss. Ein weiterer Vorteil besteht darin, dass eine Zusammensetzung mit geringer Viskosität bei der Applikation ein nur geringes Fremdkörpergefühl im Auge erzeugt und für den Patienten eine angenehmere Verträglichkeit bereitstellt. Zudem lassen sich Zusammensetzungen mit geringerer Viskosität leichter aus Dosier-Behältnissen entnehmen und einfacher applizieren.

Ein besonderer Vorteil der erfindungsgemäßen Zusammensetzung ist, dass die Stabilität der ophthalmischen Zusammensetzung ohne Zusatz von Emulgatoren erzielt werden kann. Zudem kann auf die Zugabe eines Puffers verzichtet werden. Somit kann erfindungsgemäß eine emulgatorfreie und pufferfreie Zusammensetzung bereitgestellt werden. Dies ermöglicht eine Verbesserung der Verträglichkeit der erfindungsgemäßen Zusammensetzung und vermeidet Reizungen oder das "Brennen" am Auge.

Gemäß einem Aspekt der vorliegenden Erfindung wird eine Verwendung von Hyaluronsäure und/oder Hyaluronat zur Stabilisierung einer mehrphasigen, emulgatorfreien Zusammensetzung, umfassend mindestens eine flüssige wässrige Phase und mindestens eine flüssige hydrophobe Phase, bereitgestellt. Gemäß einer Ausführungsform der vorliegenden Erfindung wird eine Verwendung von Hyaluronsäure und/oder Hyaluronat zur Stabilisierung einer tropfbaren, mehrphasigen, emulgatorfreien, pufferfreien Zusammensetzung, umfassend mindestens eine flüssige wässrige Phase und mindestens eine flüssige hydrophobe Phase, bereitgestellt.

Weitere Bestandteile

Zusätzlich zu den oben beschriebenen Komponenten, kann die ophthalmische Zusammensetzung der vorliegenden Erfindung weitere Bestandteile enthalten.

Gemäß einer Ausführungsform umfasst die Zusammensetzung zusätzlich mindestens eine polymere, gelbildende Komponente. Hierdurch kann beispielsweise die Viskosität der Zusammensetzung gezielt gesteuert werden. Zudem kann durch eine polymere, gelbildende Komponente die Adsorbierbarkeit der Zusammensetzung an die Muzinschicht des Tränenfilms verbessert und so die Verweildauer der Zusammensetzung im Auge verlängert werden. Die Schmiereigenschaften und/oder das Wasserbindevermögen der Zusammensetzung können durch Anwesenheit einer polymeren, gelbildenden Komponente ebenfalls verbessert werden.

Vorzugsweise kann die polymere, gelbildende Komponente mindestens eine Polyacrylsäure und/oder mindestens ein polymeres Acrylsäurederivat umfassen.

Bevorzugte Polyacrylsäuren oder polymere Acrylsäurederivate weisen beispielsweise ein Molekulargewicht $M_w$ im Bereich von etwa 3 bis 5 Millionen g/mol auf. Besonders bevorzugt verwendbare Polyacrylsäuren oder polymere Acrylsäurederivate sind die unter dem INCI-Bezeichnung Carbomer bezeichneten quervernetzten Acrylsäurepolymere, vorzugsweise hydrophobisch modifizierte quervernetzte Acrylsäurepolymere. Bevorzugte Carbomere sind unter dem Handelsnamen Carbopol® beispielsweise bei der Firma The Lubrizol Corporation, U.S.A., erhältlich. Besonders bevorzugte Carbomere sind Acrylsäure-Homopolymere, beispielsweise Carbopol®-Homopolymere, besonders bevorzugt verwendbar ist Carbopol® 980 NF, weiterhin bevorzugt verwendbar ist Carbopol® 940 NF. Die Begriffe Polyacrylsäure und polymere Acrylsäure werden im Sinne dieser Anmeldung synonym verwendet. Gemäß einer bevorzugten Ausführungsform umfasst die erfindungsgemäße Zusammensetzung mindestens ein Carbomer.

Gemäß einer Ausführungsform umfasst die Zusammensetzung die polymere, gelbildende Komponente in einer Menge von 0,01 bis 5 Gew.-%, bezogen auf das Gesamtgewicht der Zusammensetzung, bevorzugt von 0,02 bis 3 Gew.-

%, bevorzugter von 0,04 bis 1 Gew.-%, und am meisten bevorzugt von 0,05 bis 0,08 Gew.-%. Gemäß einer anderen Ausführungsform umfasst die Zusammensetzung mindestens eine Polyacrylsäure und/oder mindestens ein polymeres Acrylsäurederivat, bevorzugt mindestens ein Carbomer, in einer Menge von 0,01 bis 5 Gew.-%, bezogen auf das Gesamtgewicht der Zusammensetzung, bevorzugt von 0,02 bis 3 Gew.-%, bevorzugter von 0,04 bis 1 Gew.-%, und am meisten bevorzugt von 0,05 bis 0,1 Gew.-%.

Gemäß einer Ausführungsform umfasst die Zusammensetzung die polymere gelbildende Komponente in einer Menge, so dass die Zusammensetzung eine Viskosität von weniger als 350 mPa·s bei 20°C aufweist, bevorzugt weniger als 250 mPa·s bei 20°C, bevorzugter weniger als 200 mPa·s bei 20°C, noch bevorzugter weniger als 150 mPa·s bei 20°C, und am meisten bevorzugt weniger als 100 mPa·s bei 20°C.

Gemäß einer Ausführungsform umfasst die erfindungsgemäße Zusammensetzung zusätzlich ein Mittel zur Einstellung der Isotonizität, d.h. ein Isotonisierungsmittel. Als Isotonisierungsmittel können Nichtelektrolyte und/oder Elektrolyte eingesetzt werden. Beispiele für zur Einstellung der Isotonizität geeignete Nichtelektrolyte sind Dextrose, Glycerin, Propylenglycol, Sorbitol, Mannitol, Harnstoff, Polyethylenglycol, oder Mischungen davon. Beispiele für zur Einstellung der Isotonizität geeignete Elektrolyte sind Borsäure, Magnesiumsulfat, Zinksulfat, Natriumchlorid, Kaliumchlorid, Calciumchlorid, Natriumsulfat, Natriumhydrogenphosphat, Trinatriumcitrat, Trinatriumphosphat, oder Mischungen davon.

Gemäß einer Ausführungsform umfasst die Zusammensetzung ein Mittel zur Einstellung der Isotonizität ausgewählt aus der Gruppe bestehend aus Dextrose, Glycerin, Propylenglycol, Sorbitol, Mannitol, Harnstoff, Polyethylenglycol, Borsäure, Magnesiumsulfat, Zinksulfat, Natriumchlorid, Kaliumchlorid, Calciumchlorid, Natriumsulfat, Natriumhydrogenphosphat, Trinatriumcitrat, Trinatriumphosphat, und Mischungen davon, und bevorzugter ausgewählt aus der Gruppe bestehend aus Dextrose, Glycerin, Propylenglycol, Sorbitol, Mannitol, Harnstoff, Polyethylenglycol, und Mischungen davon. Gemäß einer bevorzugten Ausführungsform ist das Mittel zur Einstellung der Isotonizität Glycerin.

Gemäß einer Ausführungsform umfasst die Zusammensetzung das Mittel zur Einstellung der Isotonizität in einer Menge, die ausreicht, um die Zusammensetzung isotonisch mit natürlicher Tränenflüssigkeit bereit zu stellen. In bevorzugten Ausführungsformen umfasst die Zusammensetzung das Mittel zur Einstellung der Isotonizität in einer Menge von 0,1 bis 10 Gew.-%, bezogen auf das Gesamtgewicht der Zusammensetzung, bevorzugt von 0,5 bis 9 Gew.-%, bevorzugter von 1 bis 8 Gew.-%, und am meisten bevorzugt von 1,5 bis 7 Gew.-%. In einer weiteren bevorzugten Ausführungsformen umfasst die Zusammensetzung Glycerin als Isotonisierungsmittel in einer Menge von 0,1 bis 10 Gew.-%, bezogen auf das Gesamtgewicht der Zusammensetzung, bevorzugt von 0,5 bis 9 Gew.-%, bevorzugter von 1 bis 5 Gew.-%, und am meisten bevorzugt von 2 bis 3 Gew.-%. Gemäß einer Ausführungsform umfasst die Zusammensetzung das Mittel zur Einstellung der Isotonizität in einer Menge so dass die Zusammensetzung eine Osmolalität von 100 bis 500 mosmol/kg aufweist, bevorzugt von 200 bis 400 mosmol/kg, und am meisten bevorzugt von 260 bis 320 mosmol/kg.

Die erfindungsgemäße Zusammensetzung kann vorzugsweise keine Arzneistoffe oder pharmazeutisch aktiven Substanzen wie antivirale Mittel, steroidale und nicht-steroidale entzündungshemmende Verbindungen oder Corticosteroide, Antibiotika, Antimykotika, Narkotika, entzündungshemmender Agenzien oder antiallergische Agenzien enthalten. "Pharmazeutisch aktive Substanzen" im Sinne der Erfindung sind insbesondere nicht Vitamin A und Vitamin E.

Gemäß einer Ausführungsform umfasst die erfindungsgemäße Zusammensetzung mindestens einen ophthalmischen Wirkstoff. Beispielsweise kann die erfindungsgemäße Zusammensetzung Arzneistoffe oder pharmazeutisch aktiven Substanzen wie antivirale Mittel, steroidale und nicht-steroidale entzündungshemmende Verbindungen oder Corticosteroide, Antibiotika, Antimykotika, Narkotika, entzündungshemmende Wirkstoffe, immunosuppressive Wirkstoffe und/oder antiallergische Wirkstoffe enthalten.

Gemäß einer Ausführungsform umfasst die Zusammensetzung zusätzlich einen immunosuppressiven Wirkstoff, bevorzugt ausgewählt aus der Gruppe bestehend aus Cyclosporin A, Azathioprin, Cyclophosphamid, Tacrolimus Hydrat, Mycophenolat-Mofetil, Mycophenolsäure, Pimecrolimus, Pimecrolimus Hydrat, Sirolimus, Sirolimus Hydrat und Mischungen davon. Gemäß einer bevorzugten Ausführungsform umfasst die Zusammensetzung zusätzlich Cyclosporin A.

Gemäß einer weiteren Ausführungsform umfasst die Zusammensetzung zusätzlich eine Vitamin A-Komponente, bevorzugt Vitamin A-palmitat.

Gemäß einer Ausführungsform umfasst die Zusammensetzung mindestens einen ophthalmischen Wirkstoff, bevorzugt eine Vitamin A-Komponente, am meisten bevorzugt Vitamin A-palmitat, und/oder ein Antioxidans, am meisten bevorzugt Vitamin E. Vorzugsweise kann der Gehalt an Vitamin A-palmitat bei 1000 I.E. Vitamin A-palmitat pro Gramm Zusammensetzung liegen, wobei vorteilhafter Weise zusätzlich 20 % Stabilitätszuschlag verwendet werden. Bevorzugt verwendbares Vitamin A-palmitat weist 1 Mio. I. E. pro Gramm Vitamin A-palmitat auf und enthält vorzugsweise als Stabilisatoren butyliertes Hydroxyanisol und/oder butyliertes Hydroxytoluol. Gemäß einer Ausführungsform umfasst die Zusammensetzung eine Vitamin A-Komponente, insbesondere Vitamin A-palmitat, in einer Menge von 0,05 bis 0,5 Gew.-%, bevorzugt von 0,08 bis 0,2 Gew.-%, und am meisten bevorzugt in einer Menge von etwa 0,1 Gew.-%, unter Berücksichtigung des Stabilitätszuschlags bei etwa 0,12 Gew.-%, bezogen auf das Gesamtgewicht der Zusammensetzung.

Besonders bevorzugt ist die Vitamin A-Komponente in Kombination mit einem Antioxidans wie Vitamin E verwendbar. Die Vitamin A-Komponente kann mit wenigstens einem Antioxidans, bevorzugt Vitamin E, besonders bevorzugt D,L-$\alpha$-Tocopherol, stabilisiert werden. Zur Stabilisierung ist besonders bevorzugt ein geringer Gehalt Antioxidans, bevorzugt Vitamin E, besonders bevorzugt D,L-$\alpha$-Tocopherol, verwendbar, beispielsweise in einer Menge von 0,002 bis 0,01 Gew.-%, bevorzugt von 0,006 bis 0,008 Gew.-%, bezogen auf das Gesamtgewicht der Zusammensetzung.

Ein weiterer Vorteil der erfindungsgemäßen Zusammensetzung ist, dass ein suspendierter bzw. in der flüssigen hydrophoben Phase gelöster Wirkstoff gleichmäßiger verteilt werden kann. Die Benetzbarkeit von Objekten, die auf die Cornea aufgelegt werden, wie Kontaktschalen oder Frontlinsen ophthalmischer Geräte, kann dadurch verbessert werden.

Der pH-Wert der erfindungsgemäßen Zusammensetzung kann mit Säuren und/oder Basen eingestellt werden. Beispielsweise kann der pH-Wert mit Borsäure und/oder Natronlauge eingestellt werden. Gemäß einer Ausführungsform umfasst die erfindungsgemäße Zusammensetzung eine Säure und/oder Base in einer Menge von 0,01 bis 1 Gew.-%, bezogen auf das Gesamtgewicht der Zusammensetzung, bevorzugt von 0,02 bis 0,5 Gew.-%, und am meisten bevorzugt von 0,02 bis 0,05 Gew.-%.

Gemäß einer Ausführungsform umfasst die Zusammensetzung Natronlauge zur Einstellung des pH-Wertes. Zur Einstellung des pH-Wertes ist eine 2 %ige bis 3%ige Lösung von Natriumhydroxid in Wasser besonders geeignet. Gemäß einer Ausführungsform umfasst die erfindungsgemäße Zusammensetzung Natriumhydroxid in einer Menge von 0,01 bis 1 Gew.-%, bezogen auf das Gesamtgewicht der Zusammensetzung, bevorzugt von 0,02 bis 0,5 Gew.-%, und am meisten bevorzugt von 0,02 bis 0,05 Gew.-%.

Die erfindungsgemäße Zusammensetzung kann weiterhin ein wasserlösliches Phosphatsalz umfassen.

Gemäß einer bevorzugten Ausführungsform der vorliegenden Erfindung ist die Zusammensetzung frei von Phosphatsalzen.

Gemäß einer Ausführungsform weist die erfindungsgemäße Zusammensetzung einen pH-Wert im Bereich von 6 bis 8 auf, bevorzugt im Bereich von 6,5 bis 7,5, und am meisten bevorzugt im Bereich von 6.8 bis 7.2. Gemäß einer beispielhaften Ausführungsform weist die erfindungsgemäße Zusammensetzung einen pH-Wert im Bereich von 6 bis 7 auf.

Die erfindungsgemäße Zusammensetzung kann weitere Komponenten enthalten, beispielsweise Konservierungsmittel, bevorzugt ausgewählt aus der Gruppe enthaltend Cetrimid, Benzododeciniumchlorid, Benzalkoniumchlorid, Thiomersal, Alexidin, und Mischungen davon, und am meisten bevorzugt Cetrimid und/oder Alexidin. "Cetrimid" ist die übliche Bezeichnung für N-Cetyl-N,N,N-trimethyl-ammoniumbromid, "Benzododeciniumchlorid" für N-Benzyl-N-dodecyl-N,N-dimethyl-ammoniumchlorid, "Benzalkoniumchlorid" für Benzyllauryldimethylammoniumchlorid, und "Thiomersal" für das Natriumsalz von 2-(Ethylmercurithio)-benzoesäure. Bei Alexidin handelt es sich im Sinne der vorliegenden Erfindung nicht um einen Emulgator, oder Alexidin ist in Mengen enthalten, dass der Stoff keine emulgierende Wirkung aufweist.

Gemäß einer Ausführungsform umfasst die erfindungsgemäße Zusammensetzung ein Konservierungsmittel in einer Menge von 0,001 bis 0,05 Gew.-%, bezogen auf das Gesamtgewicht der Zusammensetzung, bevorzugt von 0,005 bis 0,01 Gew.-%.

In einer besonders bevorzugten Ausführungsform ist die erfindungsgemäße Zusammensetzung frei von Konservierungsmitteln.

Gemäß einer Ausführungsform der vorliegenden Erfindung umfasst die tropfbare, lagerungsstabile, mehrphasige, ophthalmische Zusammensetzung Hyaluronsäure und/oder ein Hyaluronat, ein ophthalmisch verträgliches Öl, mindestens eine polymere, gelbildende Komponente, ein Mittel zur Einstellung der Isotonizität, eine Base und Wasser. Gemäß einer weiteren Ausführungsform der vorliegenden Erfindung umfasst die tropfbare, lagerungsstabile, mehrphasige, ophthalmische Zusammensetzung Hyaluronsäure und/oder ein Hyaluronat, ein mittelkettiges Triglycerid, mindestens eine Polyacrylsäure und/oder mindestens ein polymeres Polyacrylsäurederivat, ein Mittel zur Einstellung der Isotonizität, eine Base und Wasser. Gemäß einer weiteren Ausführungsform der vorliegenden Erfindung umfasst die tropfbare, lagerungsstabile, mehrphasige, ophthalmische Zusammensetzung Hyaluronsäure und/oder ein Hyaluronat, bevorzugt ein Hyaluronat, ein mittelkettiges Triglycerid, ein Carbomer, ein Mittel zur Einstellung der Isotonizität ausgewählt aus der Gruppe bestehend aus Dextrose, Glycerin, Propylenglycol, Sorbitol, Mannitol, Harnstoff, Polyethylenglycol, und Mischungen davon, eine Base, bevorzugt Natriumhydroxid, und Wasser. Gemäß einer Ausführungsform ist die erfindungsgemäße Zusammensetzung frei von Phosphatsalzen und Konservierungsmitteln.

Gemäß einer Ausführungsform der vorliegenden Erfindung umfasst die tropfbare, lagerungsstabile, mehrphasige, ophthalmische Zusammensetzung, bezogen auf das Gesamtgewicht der Zusammensetzung, die folgenden Stoffe: Hyaluronsäure und/oder ein Hyaluronat, bevorzugt ein Hyaluronat, in einer Menge von 0,001 bis 5 Gew.-%, bevorzugt von 0,01 bis 2 Gew.-%, bevorzugter von 0,1 bis 1,5 Gew.-%, noch bevorzugter von 0,15 bis 1 Gew.-%, und am meisten bevorzugt von 0,2 bis 0,8 Gew.-%, ein ophthalmisch verträgliches Öl, bevorzugt ein mittelkettiges Triglycerid, in einer Menge von 0,05 bis 10 Gew.-%, bevorzugt von 0,1 bis 5 Gew.-%, bevorzugter von 0,15 bis 2 Gew.-%, und am meisten bevorzugt von 0,2 bis 1,5 Gew.-%, mindestens eine polymere, gelbildende Komponente, bevorzugt eine Polyacrylsäure und/oder ein polymeres Polyacrylsäurederivat, bevorzugter ein Carbomer, in einer Menge von 0,01 bis 5 Gew.-%, bevorzugt von 0,02 bis 3 Gew.-%, bevorzugter von 0,04 bis 1 Gew.-%, und am meisten bevorzugt von 0,05 bis 0,1 Gew.-%, ein Mittel zur Einstellung der Isotonizität, bevorzugt ausgewählt aus der Gruppe bestehend aus Dextrose, Glycerin, Propylenglycol, Sorbitol, Mannitol, Harnstoff, Polyethylenglycol, und Mischungen davon, bevorzugter Glycerin, in einer Menge von 0,1 bis 10 Gew.-%, bevorzugt von 0,5 bis 9 Gew.-%, bevorzugter von 1 bis 8 Gew.-%, und am meisten bevorzugt von 1,5 bis 7 Gew.-%, eine Base, bevorzugt Natriumhydroxid, in einer Menge von 0,01 bis 1 Gew.-%, bezogen auf das Gesamtgewicht der Zusammensetzung, bevorzugt von 0,02 bis 0,5 Gew.-%, und am meisten bevorzugt von 0,02 bis 0,05 Gew.-%, und Wasser.

Gemäß einer Ausführungsform umfasst die tropfbare, lagerungsstabile, mehrphasige, ophthalmische Zusammensetzung, bezogen auf das Gesamtgewicht der Zusammensetzung, die folgenden Stoffe:

0,15 bis 0,3 Gew.-% Natrium-Hyaluronat,
0,15 bis 0,25 Gew.-% mittelkettige Triglyceride,
0,05 bis 0,1 Gew.-% Carbomer,
1,5 bis 7 Gew.-% Mittel zur Einstellung der Isotonizität, bevorzugt Glycerin, Natriumhydroxid zur pH Einstellung, bevorzugt in einer Menge von 0,02 bis
0,05 Gew.-%, und
q.s. ad 100 Gew.-% Wasser.

Der Ausdruck "q.s. ad 100 Gew.-% Wasser" bedeutet im Sinne der vorliegenden Erfindung, dass so viel Wasser zugegeben wird, wie benötigt wird, um eine Gesamtmenge von 100 Gew.-% zu erreichen.

Gemäß einer bevorzugten Ausführungsform enthält die erfindungsgemäße Zusammensetzung neben den oben genannten Stoffen keine weiteren Bestandteile.

Eigenschaften, Herstellung und Verwendung

Gemäß der vorliegenden Erfindung wird eine tropfbare, lagerungsstabile, mehrphasige, ophthalmische Zusammensetzung bereitgestellt, enthaltend mindestens eine flüssige wässrige Phase und mindestens eine flüssige hydrophobe Phase, dadurch gekennzeichnet, dass die Zusammensetzung emulgatorfrei ist, die Zusammensetzung pufferfrei ist, und die Zusammensetzung Hyaluronsäure und/oder Hyaluronat enthält.

Gemäß einer Ausführungsform weist die erfindungsgemäße Zusammensetzung eine Viskosität von weniger als 350 mPa·s bei 20°C auf, bevorzugt weniger als 250 mPa·s bei 20°C, bevorzugter weniger als 200 mPa·s bei 20°C, noch bevorzugter weniger als 150 mPa·s bei 20°C, und am meisten bevorzugt weniger als 100 mPa·s bei 20°C. Gemäß der vorliegenden Erfindung wird die Viskosität nach dem Kegel-Platte-Verfahren unter Verwendung eines Wells-Brookfield Kegel-Platte-Viskosimeters des Typs DV-III+ (Brookfield Engineering Laboratories Vertriebs GmbH, Deutschland) unter Verwendung einer CP51 Spindel bei 5 U/min und 20°C bestimmt.

Die erfindungsgemäße Zusammensetzung lässt sich unproblematisch aseptisch herstellen und ist hervorragend verträglich. Geeignete Verfahren zur Herstellung von mehrphasigen Zusammensetzungen sind dem Fachmann bekannt. Die Herstellung der erfindungsgemäßen Zusammensetzung erfolgt vorzugsweise in einem mehrstufigen Verfahren. Insbesondere wird der flüssigen wässrigen Phase der erfindungsgemäßen Zusammensetzung Hyaluronsäure und/oder Hyaluronat zugesetzt. Es versteht sich von selbst, dass die ophthalmische Zusammensetzung zur Verwendung vorzugsweise steril vorliegt, so dass sterile Stoffe unter sterilen Bedingungen verwendet werden, oder die Zusammensetzung nach der Einarbeitung der Stoffe sterilisiert wird.

Gemäß einem weiteren Aspekt der vorliegenden Erfindung wird ein Verfahren zur Herstellung einer tropfbaren, lagerungsstabilen, mehrphasigen, ophthalmischen Zusammensetzung bereitgestellt, dadurch gekennzeichnet, dass eine flüssige hydrophobe Phase homogen in einer kontinuierlichen flüssigen wässrigen Phase dispergiert wird, wobei die flüssige wässrige Phase Hyaluronsäure und/oder Hyaluronat umfasst.

Bei einem bevorzugten Verfahren zur Herstellung der erfindungsgemäßen Zusammensetzung wird zunächst Hyaluronsäure und/oder Hyaluronat in der flüssigen wässrigen Phase gelöst, bevorzugt unter aseptischen Bedingungen. In dieser wässrigen flüssigen Phase wird dann die hydrophobe flüssige Phase, vorzugsweise unter aseptischen Bedingungen, dispergiert. Bevorzugt wird bis zur vollständigen Homogenisierung gerührt. Der Durchmesser der so erzeugten Tröpfchen der hydrophoben flüssigen Phase in der Dispersion liegt vorzugsweise bei weniger 100 μm. Gemäß einer bevorzugten Ausführungsform wird so lange homogenisiert bis Tröpfchen mit einem Durchmesser von 1 bis 30 μm erhalten werden, vorzugsweise von 5 bis 15 μm. Die erhaltene, bevorzugt sterile, Zusammensetzung kann anschließend in üblicher Weise konfektioniert werden.

Gemäß einer weiteren Ausführungsform kann der flüssigen wässrigen Phase mindestens eine polymere, gelbildende Komponente zugesetzt werden. Hierdurch kann eine dreiphasige Zusammensetzung bereitgestellt werden, die zusätzlich zu der flüssigen wässrigen und der flüssigen hydrophoben Phase noch eine Gel-Phase bzw. Muzin-Phase enthält. Des Weiteren können der flüssigen wässrigen Phase ein Mittel zur Isotonisierung und/oder andere der oben beschriebenen, weiteren Bestandteile zugesetzt werden.

Gemäß einer beispielhaften Ausführungsform wird zunächst eine Suspension aus mindestens einer Polyacrylsäure und/oder mindestens einem Acrylsäurederivat hergestellt, bevorzugt unter aseptischen Bedingungen. Zu dieser Suspension wird anschließend eine wässrige sterilfiltrierte Lösung gegeben, die Hyaluronsäure und/oder Hyaluronat sowie gegebenenfalls ein Mittel zur Isotonisierung enthält, wobei unter Verwendung von bevorzugt sterilfiltiertem Stickstoff oder Druckluft als Druckgas gearbeitet werden kann. Nach sorgfältigem Durchmischen werden dann durch Zugabe einer geeigneten Base, vorzugsweise einer sterilen 2 %igen bis 3 %igen Natriumhydroxidlösung, die Carboxylgruppen der mindestens einer Polyacrylsäure und/oder mindestens einem Acrylsäurederivat neutralisiert, die Gelbildung der mindestens einer Polyacrylsäure und/oder mindestens einem Acrylsäurederivat eingeleitet, und es wird vorzugsweise bis zur Homogenität des Gels weitergerührt. In das hergestellte sterile Hydrogel wird dann die flüssige hydrophobe Phase unter aseptischen Bedingungen eingearbeitet.

Des Weiteren kann dem so hergestellten sterilen Gel ein ophthalmischer Wirkstoff, z.B. Vitamin A-palmitat, zugesetzt werden. Gemäß einer beispielhaften Ausführungsform wird der Wirkstoff unter aseptischen Bedingungen zugesetzt und homogen eingemischt. Zum Beispiel kann eine Vitamin A-Komponente und die vorzugsweise vorhandene sehr viel geringere Menge eines Antioxidans in der hydrophoben Phase gelöst und dann steril filtriert werden. Die sterile hydrophobe wirkstoffhaltige Phase kann dann unter Rühren in das Gel eingearbeitet werden.

Die erfindungsgemäße Zusammensetzung eignet sich besonders zur Behandlung von Krankheiten oder Zuständen des Auges oder der das Auge umgebenden oder damit zusammenhängenden Organe oder Gewebe. Insbesondere ist die ophthalmische Zusammensetzung zur Linderung der Beschwerden bei trockenem Auge und/oder für die symptomatische Behandlung des trockenen Auges geeignet.

Gemäß einem weiteren Aspekt der vorliegenden Erfindung wird daher eine tropfbare, lagerungsstabile, mehrphasige, ophthalmische Zusammensetzung zur Verwendung als Arzneimittel bereitgestellt, wobei die Zusammensetzung mindestens eine flüssige wässrige Phase und mindestens eine flüssige hydrophobe Phase enthält, und dadurch gekennzeichnet ist, dass sie emulgatorfrei und pufferfrei ist und Hyaluronsäure und/oder Hyaluronat enthält. Gemäß einer Ausführungsform wird eine tropfbare, lagerungsstabile, mehrphasige, ophthalmische Zusammensetzung gemäß der vorliegenden Erfindung, zur Verwendung bei der Behandlung von Krankheiten und Fehlfunktionen des Auges oder der das Auge umgebenden oder damit zusammenhängenden Organe und Gewebe, bevorzugt zur Verwendung bei der Behandlung des trockenen Auges, wobei die Zusammensetzung mindestens eine flüssige wässrige Phase und mindestens eine flüssige hydrophobe Phase enthält, und dadurch gekennzeichnet ist, dass sie emulgatorfrei und pufferfrei ist und Hyaluronsäure und/oder Hyaluronat enthält.

Auf Grund der Abwesenheit von augenreizenden Stoffen wie z.B. Emulgatoren oder Puffersubstanzen, eignet sich die erfindungsgemäße ophthalmische Zusammensetzung zur Langzeitanwendung bei trockenem Auge, beispielsweise als Tränenersatzmittel. Es wurde überraschend gefunden, dass schon innerhalb weniger Minuten nach der Applikation der erfindungsgemäßen Zusammensetzung eine deutliche Besserung der Beschwerden wie Trockenheitsgefühl am Auge, aber auch Brennen, Jucken, Rötungen oder Schwellungen, bewirkt werden kann. Von besonderem Vorteil ist, dass diese Beschwerden nachhaltig durch die erfindungsgemäße Zusammensetzung gelindert werden können. Die konservierungsmittelfreie und/oder phosphatsalzfreie erfindungsgemäße Zusammensetzung ist für eine Langzeitanwendung besonders gut geeignet.

Für die Applikation kann die erfindungsgemäße Zusammensetzung in geeignete Behältnisse abgefüllt werden, die vorzugsweise so gestaltet sind, dass der Inhalt auf das Auge aufgebracht werden kann.

Gemäß einem weiteren Aspekt wird ein Behältnis enthaltend eine Zusammensetzung gemäß der vorliegenden Erfindung bereitgestellt, wobei das Behältnis ein Einmaldosen-Behältnis oder ein Mehrdosen-Behältnis ist, bevorzugt eine Ophtiole, eine Ein-Dosis-Ophtiole, oder ein Applikationssystem, bevorzugter ein pumpenbasiertes Applikationssystem oder ein Tip-Seal-Applikationssystem.

Gemäß einer Ausführungsform der vorliegenden Erfindung wird die erfindungsgemäße Zusammensetzung in Form eines Einmaldosen-Behältnisses bereitgestellt. Einmaldosis-Behältnisse sind im Stand der Technik beispielsweise bekannt unter der Bezeichnung Eindosis-Ophtiole®, und können bevorzugt aus einem Polyethylen wie z.B. LDPE (low density polyethylen, Polyethylen niederer Dichte) bestehen. In einer besonders bevorzugten Ausführungsform wird die erfindungsgemäßen Zusammensetzung in einem Einmaldosis-Behältnis frei von Konservierungsmitteln bereitgestellt.

Alternativ kann das Behältnis, welches die erfindungsgemäße Zusammensetzung umfasst, auch ein Mehrdosen-Behältnis sein. Beispielsweise kann das Behältnis eine Tropflasche sein. Geeignete Tropfflaschen sind im Stand der Technik bekannt, z.B. unter der Bezeichnung Ophtiole®, und können aus Polyethylen bestehen, bevorzugt aus HDPE (high density polyethyle,n Polyethylen hoher Dichte) oder LDPE (low density polyethylen, Polyethylen niederer Dichte).

Der Mehrdosen-Behältnis kann auch ein Applikationssystem sein, wie z.B. ein pumpenbasiertes Applikationssystem oder ein Tip-Seal-System. Beispiele für pumpenbasierte Applikationssysteme sind Augensprays sowie Augentropfenpumpen, wie z.B. das COMOD®-System oder das 3K®-System, beides hergestellt von der Firma Ursatec Verpackung GmbH. Beispiele für Tip-Seal-Systeme sind das Ophthalmic Squeeze Dispenser (OSD®)-System der Firma Aptargroup, Inc., oder das Novelia®-System der Firma Nemera.

Umfang und Gegenstand der vorliegenden Erfindung werden durch die nachfolgenden Beispiele anhand von ausgewählten Ausführungsformen veranschaulicht, die jedoch nicht als einschränkend zu verstehen sind.

**Beispiele**

**1. Messmethoden**

pH-Wert

Der pH-Wert der hergestellten, ophthalmischen Zusammensetzungen wurde bei einer Temperatur von 20°C mit einem Knick pH-Meter 765 oder Knick pH-Meter 766 (Knick Elektronische Messgeräte GmbH & Co. KG, Deutschland) gemessen.

Osmolarität

Die Osmolarität der hergestellten, ophthalmischen Zusammensetzungen wurde bei einer Temperatur von 20°C mit einem Mikro-Osmometer gemessen (Hermann Röbling Messtechnik, Deutschland).

Viskosität

Die Viskosität der hergestellten, ophthalmischen Zusammensetzungen wurde nach dem Kegel-Platte-Verfahren unter Verwendung eines Wells-Brookfield Kegel-Platte-Viskosimeters des Typs DV-III+ (Brookfield Engineering Laboratories Vertriebs GmbH, Deutschland) mit einer CP51 Spindel bei 5 U/min und 20°C bestimmt.

Messung der Tröpfchengröße

Der Durchmesser der Tröpfchen der flüssigen hydrophoben Phase wurde mikroskopisch mit dem Mikroskop Axio Imager M1 (Carl Zeiss AG, Deutschland) bestimmt.

Tropfversuch (Priming)

Die Tropfversuche wurden manuell durch senkrechte Betätigung eines pumpenbasierten Applikationssystems (3K®-System, Ursatec Verpackung GmbH, Deutschland), durchgeführt. Dabei wurde die erforderliche Anzahl an Betätigungen bestimmt, bis ein Tropfen mittlerer Tropfengröße vom Applikationssystem abgegeben wurde.

Stabilitätstest

Die Lagerungsstabilität der hergestellten, ophthalmischen Zusammensetzungen wurde durch Zentrifugieren der Zusammensetzung bei einer Geschwindigkeit von 5700 Umdrehungen pro Minute bei 20°C über einen Zeitraum von 60 Minuten bestimmt. Für diese Untersuchungen wurde eine Laborzentrifuge Multifuge 1 S (Kendro Laboratory Products GmbH, Deutschland) verwendet.

Nach Beendigung des Zentrifugierens wurde die Zusammensetzung visuell begutachtet und wie folgt bewertet:

OK: Optisch keine Trennung oder Trübungsunterschiede erkennbar.
ET: Eintrübung von unten erkennbar.

**2. Stoffe**

HA:          Natrium-Hyaluronat (Bloomage Freda Biopharm Co., Ltd., China).
MCT:         Capryl-Caprinsäure-Triglycerid (Myritol 318, BASF AG, Deutschland).
Carbomer:    Carbopol 980 NF (Lubrizol Advanced Materials Europe BVBA, Belgium).
IM:          Isotonisierungsmittel; folgende Stoffe wurden eingesetzt: Glycerin, Sorbitol, Mannitol, Glucose, Propylenglycol, Harnstoff, PEG 300.

**3. Beispiel**

Es wurden ophthalmische Zusammensetzungen, bezeichnet mit Probe 1 bis 9 nach dem folgenden Verfahren unter Verwendung der in Tabelle 1 genannten Stoffe und Mengen hergestellt.

Im ersten Schritt der Herstellung wurde Carbomer in Wasser suspendiert und anschließend autoklaviert. Im zweiten Schritt wurde eine sterile wässrige Lösung, bestehend aus Wasser, Natrium-Hyaluronat und einem Isotonisierungsmittel hergestellt und der sterilen Carbomersuspension aseptisch zugegeben. Nachfolgend wurde ein pH-Wert von 6 bis 7, sofern notwendig durch Zugabe steriler NaOH-Lösung eingestellt, und die flüssige hydrophobe Phase unter Homogenisierung zugegeben. Die fertiggestellte Zusammensetzung wurde aseptisch in ein Primärpackmittel abgefüllt.

Die in Tabelle 2 zusammengefassten Ergebnisse zeigen, dass alle erfindungsgemäßen Proben 2 bis 9 den Stabilitätstest bestehen und daher lagerungsstabil sind. Dagegen zeigt die Vergleichsprobe 1 ohne Hyaluronat nach der Durchführung des Stabilitätstests eine Eintrübung von unten, was auf eine Entmischung der Phasen hinweist.

Tabelle 1: Stoffe und Mengen der hergestellten Proben 1 bis 9 (Vgl.: Vergleichsprobe).

| Stoff | Probe 1 (Vgl.) | Probe 2 | Probe 3 | Probe 4 | Probe 5 | Probe 6 | Probe 7 | Probe 8 | Probe 9 |
|---|---|---|---|---|---|---|---|---|---|
| HA [mg/g] | -- | 2,4 | 2,4 | 2,4 | 2,4 | 2,4 | 2,4 | 2,4 | 1,7 |
| MCT [mg/g] | 2,0 | 2,0 | 2,0 | 2,0 | 2,0 | 2,0 | 2,0 | 2,0 | 2,0 |
| Carbomer [mg/g] | 0,625 | 0,625 | 0,625 | 0,625 | 0,625 | 0,625 | 0,625 | 0,625 | 0,625 |
| NaOH | q.s. pH 6-7 | q.s. pH 6-7 | q.s. pH 6-7 | q.s. pH 6-7 | q.s. pH 6-7 | q.s. pH 6-7 | q.s. pH 6-7 | q.s. pH 6-7 | q.s. pH 6-7 |
| IM [mg/g] | Glycerin 29,0 | Glycerin 29,0 | Sorbitol 50,0 | Mannitol 49,0 | Glucose 52,8 | Propylenglycol 20,0 | Harnstoff 16,3 | PEG 300 65,0 | Glycerin 29,0 |
| Wasser | ad 1 g | ad 1 g | ad 1 g | ad 1 g | ad 1 g | ad 1 g | ad 1 g | ad 1 g | ad 1 g |

Tabelle 2: Eigenschaften der hergestellten Proben 1 bis 9 (Vgl.: Vergleichsprobe).

| | Probe 1 (Vgl.) | Probe 2 | Probe 3 | Probe 4 | Probe 5 | Probe 6 | Probe 7 | Probe 8 | Probe 9 |
|---|---|---|---|---|---|---|---|---|---|
| pH-Wert | 7,3 | 7,1 | 7,1 | 7,1 | 7,2 | 7,3 | 7,1 | 7,7 | 7,0 |
| Osmolarität [mosmol/kg] | 287 | 269 | 294 | 283 | 286 | 270 | 271 | 274 | 287 |
| Viskosität [mPa·s] | 123 | 202 | 209 | 194 | 183 | 184 | 174 | 199 | 166 |
| Tröpfchengröße [$\mu$m] | 9-12 | 9-11 | 11-12 | 7-10 | 8-11 | 9-10 | 8-10 | 9-11 | 5-10 |
| Tropfversuch (Priming) | 8 | 10 | 10 | 18 | 18 | 10 | 15 | 10 | 10 |
| Stabilitätstest | ET | OK | OK | OK | OK | OK | OK | OK | OK |

**Patentansprüche**

1. Tropfbare, lagerungsstabile, mehrphasige, ophthalmische Zusammensetzung, enthaltend mindestens eine flüssige wässrige Phase und mindestens eine flüssige hydrophobe Phase, **dadurch gekennzeichnet, dass**
die Zusammensetzung emulgatorfrei ist,
die Zusammensetzung pufferfrei ist, und
die Zusammensetzung Hyaluronsäure und/oder Hyaluronat enthält.

2. Zusammensetzung nach Anspruch 1, **dadurch gekennzeichnet, dass** die Zusammensetzung eine Viskosität von weniger als 350 mPa·s bei 20°C aufweist, bevorzugt weniger als 250 mPa·s bei 20°C, bevorzugter weniger als 200 mPa·s bei 20°C, noch bevorzugter weniger als 150 mPa·s bei 20°C, und am meisten bevorzugt weniger als 100 mPa·s bei 20°C.

3. Zusammensetzung nach einem der vorherigen Ansprüche, **dadurch gekennzeichnet, dass** die Zusammensetzung Hyaluronsäure und/oder Hyaluronat in einer Menge von mindestens 0,001 Gew.-%, bezogen auf das Gesamtgewicht der Zusammensetzung, enthält, bevorzugt in einer Menge von mindestens 0,01 Gew.-%, bevorzugter in einer Menge von mindestens 0,1 Gew.-%, noch bevorzugter in einer Menge von mindestens 0,15 Gew.-%, und am meisten bevorzugt in einer Menge von mindestens 0,2 Gew.-%.

4. Zusammensetzung nach einem der vorherigen Ansprüche, **dadurch gekennzeichnet, dass** die Hyaluronsäure und/oder das Hyaluronat ein Molekulargewicht $M_w$ von 50 000 bis 10 000 000 g/mol aufweist, bevorzugt von 100 000 bis 5 000 000 g/mol, und am meisten bevorzugt von 250 000 bis 1 000 000 g/mol.

5. Zusammensetzung nach einem der vorherigen Ansprüche, **dadurch gekennzeichnet, dass** das Hyaluronat ausgewählt ist aus der Gruppe bestehend aus Natrium-Hyaluronat, Kalium-Hyaluronat, Zink-Hyaluronat und Mischungen davon, bevorzugt ist das Hyaluronat Natrium-Hyaluronat.

6. Zusammensetzung nach einem der vorherigen Ansprüche, **dadurch gekennzeichnet, dass** die Zusammensetzung die flüssige wässrige Phase als kontinuierliche Phase und die flüssige hydrophobe Phase als darin dispergierte Tröpfchen enthält.

7. Zusammensetzung nach einem der vorherigen Ansprüche, **dadurch gekennzeichnet, dass** die Zusammensetzung mindestens eine polymere, gelbildende Komponente umfasst, bevorzugt mindestens eine Polyacrylsäure und/oder mindestens ein polymeres Acrylsäurederivat, und am meisten bevorzugt mindestens ein Carbomer.

8. Zusammensetzung nach einem der vorherigen Ansprüche, **dadurch gekennzeichnet, dass** die flüssige hydrophobe Phase ein ophthalmisch verträgliches Öl umfasst, bevorzugt ein Triglycerid, und am meisten bevorzugt ein mittelkettiges Triglycerid.

9. Zusammensetzung nach einem der vorherigen Ansprüche, **dadurch gekennzeichnet, dass** die Zusammensetzung ein Mittel zur Einstellung der Isotonizität umfasst, bevorzugt ausgewählt aus der Gruppe bestehend aus Dextrose, Glycerin, Propylenglycol, Sorbitol, Mannitol, Harnstoff, Polyethylenglycol, Borsäure, Magnesiumsulfat, Zinksulfat, Natriumchlorid, Kaliumchlorid, Calciumchlorid, Natriumsulfat, Natriumhydrogenphosphat, Trinatriumcitrat, Trinatriumphosphat, und Mischungen davon, bevorzugter ausgewählt aus der Gruppe bestehend aus Dextrose, Glycerin, Propylenglycol, Sorbitol, Mannitol, Harnstoff, Polyethylenglycol, und am meisten bevorzugt Glycerin.

10. Zusammensetzung nach einem der vorherigen Ansprüche, **dadurch gekennzeichnet, dass** die Zusammensetzung, bezogen auf das Gesamtgewicht der Zusammensetzung, die folgenden Stoffe umfasst:

0,15 bis 0,3 Gew.-% Natrium-Hyaluronat,
0,15 bis 0,25 Gew.-% mittelkettige Triglyceride,
0,05 bis 0,1 Gew.-% Carbomer,
1,5 bis 7 Gew.-% Mittel zur Einstellung der Isotonizität, bevorzugt Glycerin, Natriumhydroxid zur pH Einstellung, und
q.s. ad 100 Gew.-% Wasser.

11. Verfahren zur Herstellung einer tropfbaren, lagerungsstabilen, mehrphasigen, ophthalmischen Zusammensetzung nach einem der Ansprüche 1 bis 10, **dadurch gekennzeichnet, dass** eine flüssige hydrophobe Phase homogen in einer kontinuierlichen flüssigen wässrigen Phase dispergiert wird, wobei die flüssige wässrige Phase Hyaluronsäure und/oder Hyaluronat umfasst.

12. Tropfbare, lagerungsstabile, mehrphasige, ophthalmische Zusammensetzung nach einem der Ansprüche 1 bis 10, zur Verwendung als Arzneimittel.

13. Tropfbare, lagerungsstabile, mehrphasige, ophthalmische Zusammensetzung nach einem der Ansprüche 1 bis 10, zur Verwendung bei der Behandlung von Krankheiten und Fehlfunktionen des Auges oder der das Auge umgebenden oder damit zusammenhängenden Organe und Gewebe, bevorzugt zur Verwendung bei der Behandlung des trockenen Auges.

14. Behältnis enthaltend eine Zusammensetzung nach einem der Ansprüche 1 bis 10, wobei das Behältnis ein Einmaldosen-Behältnis oder ein Mehrdosen-Behältnis ist, bevorzugt eine Ophtiole, eine Ein-Dosis-Ophtiole, oder ein Applikationssystem, bevorzugter ein pumpenbasiertes Applikationssystem oder ein Tip-Seal-Applikationssystem.

15. Verwendung von Hyaluronsäure und/oder Hyaluronat zur Stabilisierung einer mehrphasigen, emulgatorfreien Zusammensetzung, umfassend mindestens eine flüssige wässrige Phase und mindestens eine flüssige hydrophobe Phase.

**Claims**

1. A storage stable, multiphase, ophthalmic composition comprising at least one liquid aqueous phase and at least one liquid hydrophobic phase, **characterized in that**
   the composition is emulsifier-free,
   the composition is buffer-free, and
   the composition comprises hyaluronic acid and/or hyaluronate.

2. The composition according to claim 1, **characterized in that** the composition exhibits a viscosity of less than 350 mPa·s at 20°C, preferably less than 250 mPa·s at 20°C, more preferably less than 200 mPa·s at 20°C, and even more preferably less 150 mPa·s at 20°C, and most preferably less than 100 mPa·s at 20°C.

3. The composition according to any of the preceding claims, **characterized in that** the composition comprises hyaluronic acid and/or hyaluronate in an amount of at least 0.001 wt.%, based on the total weight of the composition, preferably an amount of at least 0.01 wt.%, more preferably an amount of at least 0.1 wt.%, even more preferably an amount of at least 0.15 wt.% and most preferably an amount of at least 0.2 wt.%.

4. The composition according to any of the preceding claims, **characterized in that** the hyaluronic acid and/or hyaluronate exhibits a molecular weight $M_w$ of from 50 000 to 10 000 000 g/mol, more preferably of from 100 000 to 5 000 000 g/mol, and most preferably of from 250 000 to 1 000 000 g/mol.

5. The composition according to any of the preceding claims, **characterized in that** the hyaluronate is selected from the group consisting of sodium hyaluronate, potassium hyaluronate, zinc hyaluronate, and mixtures thereof, preferably the hyaluronate is sodium hyaluronate.

6. The composition according to any of the preceding claims, **characterized in that** the composition comprises the liquid aqueous phase as continuous phase, and the liquid hydrophobic phase as droplets dispersed therein.

7. The composition according to any of the preceding claims, **characterized in that** the composition comprises at least one polymeric gel-forming component, preferably at least one polyacrylic acid and/or a polymeric polyacrylic acid derivative, and most preferably a carbomer.

8. The composition according to any of the preceding claims, **characterized in that** the liquid hydrophobic phase comprises an ophthalmic acceptable oil, preferably a triglyceride, and most preferably a medium-chain triglyceride.

9. The composition according to any of the preceding claims, **characterized in that** the composition comprises an agent to adjust the isotonicity, preferably selected from the group of dextrose, glycerin, propylene glycol, sorbitol, mannitol, urea, polyethylene glycol, boric acid, magnesium sulfate, zinc sulfate, sodium chloride, potassium chloride, calcium chloride, sodium sulfate, sodium hydrogen phosphate, trisodium citrate, trisodium phosphate, and mixtures thereof, preferably selected from the group of dextrose, glycerin, propylene glycol, sorbitol, mannitol, urea, polyethylene glycol, and most preferably glycerin.

10. The composition according to any of the preceding claims, **characterized in that** the composition comprises the following substances, based on the total weight of the composition:

    0.15 to 0.3 wt.% sodium hyaluronate,
    0.15 to 0.25 wt.% medium-chain triglycerides,
    0.05 to 0.1 wt.% carbomer,
    1.5 to 7 wt.% agent to adjust the isotonicity, preferably glycerin,
    sodium hydroxide for pH adjustment, and
    q.s. ad 100 wt.% water.

11. A process for the preparation of a drop-forming, storage stable, multiphase, ophthalmic composition according to any one of claims 1 to 10 **characterized in that** a liquid hydrophobic phase is dispersed homogenously in a continuous liquid aqueous phase, wherein the liquid aqueous phase comprises hyaluronic acid and/or hyaluronat.

12. A drop-forming, storage stable, multiphase, ophthalmic composition according to any one of claims 1 to 10, for use as a medicament.

13. A drop-forming, storage stable, multiphase ophthalmic composition according to any one of claims 1 to 10 for use in treating diseases or conditions of the eye or the organs or tissues surrounding the eye or being connected therewith, and preferably for use in treating dry eye.

14. A container comprising a composition according to any one of claims 1 to 10, wherein the container is a single-dose container or a multi-dose container, preferably an ophtiole, a single-dose ophtiole or an application system, preferably a pump-based application system or a tip-seal application system.

15. Use of hyaluronic acid and/or hyaluronate for the stabilization of a multiphase, emulsifier-free composition comprising at least one liquid aqueous phase and at least one liquid hydrophobic phase.

**Revendications**

1. Composition ophtalmique polyphasique qui est stable au stockage, qui peut être appliquée en gouttes et qui contient au moins une phase aqueuse liquide et au moins une phase hydrophobe liquide, **caractérisée en ce que**

ladite composition est exempte d'émulsifiants,
ladite composition est exempte de tampons, et
ladite composition contient de l'acide hyaluronique et/ou de l'hyaluronate.

2. Composition selon la revendication 1, **caractérisée en ce que** ladite composition présente une viscosité inférieure à 350 mPa•s à 20 °C, de préférence inférieure à 250 mPa•s à 20 °C, de manière plus préférée inférieure à 200 mPa•s à 20 °C, de manière encore plus préférée inférieure à 150 mPa•s à 20 °C et, avec la plus grande préférence, inférieure à 100 mPa•s à 20 °C.

3. Composition selon l'une des revendications précédentes, **caractérisée en ce que** ladite composition contient de l'acide hyaluronique et/ou de l'hyaluronate dans une quantité d'au moins 0,001 % en poids, par rapport au poids total de la composition, de préférence dans une quantité d'au moins 0,01 % en poids, de manière plus préférée dans une quantité d'au moins 0,1 % en poids, de manière encore plus préférée dans une quantité d'au moins 0,15 % en poids et, avec la plus grande préférence, dans une quantité d'au moins 0,2 % en poids.

4. Composition selon l'une des revendications précédentes, **caractérisée en ce que** ledit acide hyaluronique et/ou ledit hyaluronate présente une masse moléculaire $M_w$ comprise entre 50 000 et 10 000 000 g/mol, de préférence comprise entre 100 000 et 5 000 000 g/mol et, avec la plus grande préférence, comprise entre 250 000 et 1 000 000 g/mol.

5. Composition selon l'une des revendications précédentes, **caractérisée en ce que** ledit hyaluronate est choisi dans le groupe constitué d'hyaluronate de sodium, d'hyaluronate de potassium, d'hyaluronate de zinc et de leurs mélanges, ledit hyaluronate étant préférentiellement de l'hyaluronate de sodium.

6. Composition selon l'une des revendications précédentes, **caractérisée en ce que** ladite composition contient ladite phase aqueuse liquide sous forme de phase continue et contient ladite phase hydrophobe liquide sous forme de gouttelettes qui y sont dispersées.

7. Composition selon l'une des revendications précédentes, **caractérisée en ce que** ladite composition comprend au moins un constituant polymère apte à former un gel, s'agissant préférentiellement d'au moins un acide polyacrylique et/ou d'au moins un dérivé polymérisé d'acide acrylique et, avec la plus grande préférence, d'au moins un carbomère.

8. Composition selon l'une des revendications précédentes, **caractérisée en ce que** ladite phase hydrophobe liquide comprend une huile ophtalmiquement acceptable, s'agissant préférentiellement d'un triglycéride et, avec la plus grande préférence, d'un triglycéride à chaîne moyenne.

9. Composition selon l'une des revendications précédentes, **caractérisée en ce que** ladite composition comprend un agent de régulation de l'isotonicité qui est préférentiellement choisi dans le groupe constitué de dextrose, glycérol, propylène glycol, sorbitol, mannitol, urée, polyéthylène glycol, acide borique, sulfate de magnésium, sulfate de zinc, chlorure de sodium, chlorure de potassium, chlorure de calcium, sulfate de sodium, hydrogénophosphate de sodium, citrate trisodique, phosphate trisodique et de leurs mélanges, de manière plus préférée choisi dans le groupe constitué de dextrose, glycérol, propylène glycol, sorbitol, mannitol, urée, polyéthylène glycol, s'agissant avec la plus grande préférence de glycérol.

10. Composition selon l'une des revendications précédentes, **caractérisée en ce que** ladite composition comprend les matières suivantes, par rapport au poids total de la composition:

0,15 à 0,3 % en poids d'hyaluronate de sodium,
0,15 à 0,25 % en poids de triglycérides à chaîne moyenne,
0,05 à 0,1 % en poids de carbomère,
1,5 à 7 % en poids d'agent de régulation de l'isotonicité, s'agissant préférentiellement de glycérol,
de l'hydroxyde de sodium pour ajuster le pH, et
q.s. ad 100 % en poids d'eau.

11. Procédé de fabrication d'une composition ophtalmique polyphasique qui est stable au stockage, qui peut être appliquée en gouttes et qui est conforme à l'une des revendications 1 à 10, **caractérisé en ce que** l'on disperse une phase hydrophobe liquide de manière homogène dans une phase aqueuse liquide, ladite phase aqueuse liquide comprenant de l'acide hyaluronique et/ou de l'hyaluronate.

**12.** Composition ophtalmique polyphasique qui est stable au stockage, qui peut être appliquée en gouttes et qui est conforme à l'une des revendications 1 à 10, destinée à l'utilisation en tant que médicament.

**13.** Composition ophtalmique polyphasique qui est stable au stockage, qui peut être appliquée en gouttes et qui est conforme à l'une des revendications 1 à 10, destinée à l'utilisation dans le traitement de maladies et de dysfonctionnements affectant les yeux ou les organes et tissues qui entourent les yeux ou qui y sont attenants, destinée préférentiellement à l'utilisation dans le traitement de la sécheresse oculaire.

**14.** Récipient contenant une composition selon l'une des revendications 1 à 10, ledit récipient étant un récipient monodose ou un récipient multidoses, s'agissant préférentiellement d'un récipient ophtalmique à usage unique, d'un récipient ophtalmique monodose à usage unique ou d'un système d'application, de manière plus préférée d'un système d'application à pompe ou d'un système d'application scellé à la pointe.

**15.** Utilisation d'acide hyaluronique et/ou d'hyaluronate pour stabiliser une composition polyphasique exempte d'émulsifiants qui comprend au moins une phase aqueuse liquide et au moins une phase hydrophobe liquide.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1913934 A1
- WO 2007012625 A1
- WO 2013122801 A1

**In der Beschreibung aufgeführte Nicht-Patentliteratur**

- *BfArM & PEI Bulletin zur Arzneimittelsicherheit,* 2013, vol. 1, 7-12